# EUROPEAN PATENT SPECIFICATION

(11) **EP 2 121 139 B1**
(45) Date of publication and mention of the grant of the patent: **10.10.2012**
(21) Application number: 08727771.1
(22) Date of filing: 16.01.2008
(51) Int. Cl.: A61K 9/48, A61K 31/166, A61K 47/10, A61K 47/12, A61K 47/18, A61K 47/26, A61K 47/48, A61K 9/107, A61K 9/00, A61K 9/16, B82Y 5/00

(54) **FORMULATIONS FOR CANCER TREATMENT**
FORMULIERUNGEN ZUR KREBSBEHANDLUNG
PRÉPARATIONS POUR LE TRAITEMENT DU CANCER

(30) Priority: 16.01.2007 US 880755 P
(43) Date of publication of application: 25.11.2009
(60) Divisional application: 11192725.7
(73) Proprietor: BiPar Sciences, Inc., Bridgewater NJ 08807 (US)
(72) Inventor: OSSOVSKAYA, Valeria, San Francisco, CA 94105 (US); LI, Lingyun, Foster City, CA 94404 (US); SHERMAN, Barry, Hillsborough, CA 94010 (US)
(74) Representative: Adamson Jones
(86) International application number: PCT/US2008/051214
(87) International publication number: WO 2008/089272

(56) References cited:
- EP-A- 1 348 432
- WO-A-98/45253
- WO-A-2007/011962
- US-A- 5 877 185
- CHAUBAL M V: "Application of formulation technologies in lead candidate selection and optimization", DRUG DISCOVERY TODAY, ELSEVIER, RAHWAY, NJ, US, vol. 9, no. 14, 1 July 2004 (2004-07-01), pages 603-609, XP004545602, ISSN: 1359-6446
- Trichard L, Duchêne D, Bochot A: "Chapter 15 "Cyclodextrins in Dispersed Systems"" In: Dodziuk H: "Cyclodextrins and Their Complexes", 1 January 2006 (2006-01-01), Wiley-VCH Verlag GmbH & Co. KG ISBN: 9783527312801 pages 423-449, * the whole document *

## Description

### FIELD OF THE INVENTION

The present invention relates to formulations useful in the treatment of cancer, viral diseases and other disease states. Specifically, the invention relates to formulations that facilitate use and bioavailability of aromatic nitrobenzamide compounds.

### BACKGROUND OF THE INVENTION

Cancer is a serious threat to modem society. Malignant cancerous growths, due to their unique characteristics, pose serious challenges for modern medicine. Their characteristics include uncontrollable cell proliferation resulting in unregulated growth of malignant tissue, an ability to invade local and even remote tissues, lack of differentiation, lack of detectable symptoms and most significantly, the lack of effective therapy and prevention.

Cancer can develop in any tissue of any organ at any age. The etiology of cancer is not clearly defined but mechanisms such as genetic susceptibility, chromosome breakage disorders, viruses, environmental factors and immunologic disorders have all been linked to a malignant cell growth and transformation. Cancer encompasses a large category of medical conditions, affecting millions of individuals worldwide. Cancer cells can arise in almost any organ and/or tissue of the body. Cancer develops when cells in a part of the body begin to grow or differentiate out of control. All cancer types begin with the out-of-control growth of abnormal cells.

There are many types of cancer, including, breast, lung, ovarian, bladder, prostate, pancreatic, cervical, and leukemia. Currently, some of the main treatments available are surgery, radiation therapy, and chemotherapy. Surgery is often a drastic measure and can have serious consequences. For example, all treatments for ovarian cancer may result in infertility. Some treatments for cervical cancer and bladder cancer may cause infertility and/or sexual dysfunction. Surgical procedures to treat pancreatic cancer may result in partial or total removal of the pancreas and can carry significant risks to the patient. Breast cancer surgery invariably involves removal of part of or the entire breast. Some surgical procedures for prostate cancer carry the risk of urinary incontinence and impotence. The procedures for lung cancer patients often have significant post-operative pain as the ribs must be cut through to access and remove the cancerous lung tissue. In addition, patients who have both lung cancer and another lung disease, such as emphysema or chronic bronchitis, typically experience an increase in their shortness of breath following the surgery.

Radiation therapy has the advantage of killing cancer cells but it also damages non-cancerous tissue at the same time. Chemotherapy involves the administration of various anti-cancer drugs to a patient but often is accompanied by adverse side effects.

Worldwide, more than 10 million people are diagnosed with cancer every year and it is estimated that this number will grow to 15 million new cases every year by 2020. Cancer causes six million deaths every year or 12% of the deaths worldwide. There remains a need for methods that can treat cancer. These methods can provide the basis for pharmaceutical compositions useful in the prevention and treatment of cancer in humans and other mammals.

Viral infections are also a serious threat to human health throughout the world. Human immunodeficiency virus (HIV) infections known as acquired immunodeficiency syndrome (AIDS), presently constitute a worldwide health hazard. HIV infections are almost always fatal due to a weakened immune-resistance, leading to opportunistic infections, malignancies and neurologic lesions.

There is no effective treatment for AIDS other than the treatment of the opportunistic infections, neoplasms and other complications. Available cytostatic (AZT) and antiviral (acyclovir) drugs are extremely toxic and cause severe adverse reactions.

Thus it would be highly desirable to have available an effective and yet nontoxic treatment of viral diseases, in particular, AIDS.

Herpes simplex virus type-1 and 2 are also wide-spread infections. They may occur in AIDS patients as one of the opportunistic infections. Type-1 HSV strain (HSV-1) commonly causes herpes labialism located on a lip, and keratitis, an inflammation of the cornea. Type-2 HSV is usually located on or around the genital area and is generally transmitted primarily by direct contact with herpetic sore or lesions. HSV-2 has been related to the development of uterine cancer.

Herpes simplex virus is very infectious and is rapidly and easily transferable by contact There is no specific therapy to this extremely painful viral infection. Current treatment of HSV infections is limited primarily to systemic administration of the above-mentioned antiviral drugs with corresponding adverse side affects.

The antiviral agents used for HSV treatment are non-selective inhibitors of HSV replication affecting the replication of normal cells as well. Therefore, when used in doses large enough to inactivate all of the active herpes viruses dormant in the sensory ganglia, these compounds may also be highly disruptive to host cell DNA replication.

Thus, it would be advantageous to have available non-toxic treatment of HSV infections.

Cytomegalovirus (CMV), a dangerous co-infection of HIV, is a subgroup of highly infectious viruses having the propensity for remaining latent in man. CMVs are very common among the adult population and as many as 90% of adults have been exposed to and experienced CMV infections. CMVs are normally present in body liquids such as blood, lymph, saliva, urine, feces, milk, etc. CMV infections may cause abortion, stillbirth, postnatal death from hemorrhage, anemia, severe hepatic or CNS damage. Particularly dangerous are CMV infections afflicting AIDS patients, where CMV may cause pulmonary, gastrointestinal or renal complications. There is no specific therapy for CMVs. Unlike HSV, CMV is resistant to acyclovir, and to other known antiviral drugs.

Thus, it would be extremely advantageous to have available a drug which would effectively inhibit CMV infections.

A series of anti-tumor drugs and anti-viral have been identified. These drugs include nitro and nitroso compounds and their metabolites, which are the subject of U.S. Pat. No. 5,464,871 issued on Nov. 7, 1995 entitled "Aromatic Nitro and Nitroso Compounds and their Metabolites Useful as Anti-viral and Anti-tumor Agents," Pat. No. 5,670,518 issued on Sept. 23, 1997 entitled "Aromatic Nitro and Nitroso Compounds and their Metabolites Useful as Anti-viral and Anti-tumor Agents," Pat. No. 6,004,978 issued on Dec. 21, 1999 entitled "Methods of Treating Cancer with Aromatic Nitro and Nitroso Compounds and their Metabolites". The use of the compounds has been described in the art as useful in treating mammary gland adenocarcinomas, mammary gland duct carcinomas, lymphocytic leukemia, Karposi's sarcoma in immuno-suppressed patients with AIDS, and neoplastic growths such as non-Hodgkin lymphoma, and primary lymphomas.

While nitrobenzamide compounds have been shown to be useful anti-tumor and anti-viral agents, the compounds tend to be poorly soluble in water. In order to maximize the bioavailability of a compound, it is often desirable for the compound to have good solubility in aqueous solution. Enhanced solubility in aqueous solution can increase bioavailability for many compound delivery modes including injection, oral ingestion, or transdermal delivery, because of the aqueous nature of the blood and other aqueous bodily fluids.

### SUMMARY OF THE INVENTION

The present invention relates generally to pharmaceutical compositions comprising the nitro compound 4-iodo-3-nitrobenzamide ("Compound (Ia)" / "BA") or a pharmaceutically acceptable salt thereof, as defined in Claims 1 and 8. More specifically, the invention relates to pharmaceutical compositions comprising the nitro compound 4-iodo-3-nitrobeozamide ("Compound (Ia)" / "BA") or a salt thereof and a cyclodextrin. Most preferred formulation is with 25% beta-cyclodextrin and 10 mM phosphate at pH 7.4.

The pharmaceutical compositions of this invention can be used to treat various cancers, including leukemia, breast cancer, ovarian cancer, lung cancer, bladder cancer, prostate cancer, pancreatic cancer, and cervical cancer, as well as other cancer types described herein.

The pharmaceutical compositions of this invention can also be used as anti-viral agents against various viruses including Human immunodeficiency virus (HIV), Herpes simplex virus (HSV), and Cytomegalovirus (CMV).

A composition of the invention can be a combination of two or more compounds described herein and/or a combination of two or more forms of a compound described herein. A pharmaceutical composition of the invention may be a composition suitable for administration to a subject.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

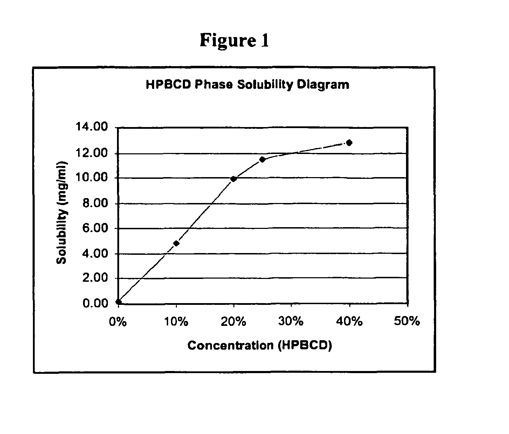

Figure 1 provides in graph form the solubility of 4-iodo-3-nitrobenzamide in water versus the concentration of hydroxypropyl-β-cyclodextrin (HPBCD; also referred to herein as "HPβCD").

### DETAILED DESCRIPTION OF THE INVENTION

A pharmaceutical composition comprising BA, or a pharmaceutically acceptable salt thereof, and a cyclodextrin that comprises one or more of: hydroxypropyl-β-cyclodextrin and sulfobutyl ether- β-cyclodextrin.

The composition is for treating a condition selected from cancer, and a viral condition.

In some embodiments, the cancer is a member of the group consisting of wherein the cancer is selected from adrenal cortical cancer, anal cancer, aplastic anemia, bile duct cancer, bladder cancer, bone cancer, bone metastasis, CNS tumors, peripheral CNS cancer, breast cancer, Castleman's Disease, cervical cancer, childhood Non-Hodgkin's lymphoma, colon and rectum cancer, endometrial cancer, esophagus cancer, Ewing's family of tumors, eye cancer, gallbladder cancer, gastrointestinal carcinoid tumors, gastrointestinal stromal tumors, gestational trophoblastic disease, hairy cell leukemia, Hodgkin's disease, Kaposi's sarcoma, kidney cancer, laryngeal and hypopharyngeal cancer, acute lymphocytic leukemia, acute myeloid leukemia, children's leukemia, chronic lymphocytic leukemia, chronic myeloid leukemia, liver cancer, lung cancer, lung carcinoid tumors, Non-Hodgkin's lymphoma, male breast cancer, malignant mesothelioma, multiple myeloma, myelodysplastic syndrome, myeloproliferative disorders, nasal cavity and paranasal cancer, nasopharyngeal cancer, neuroblastoma, oral cavity and oropharyngeal cancer, osteosarcoma, ovarian cancer, pancreatic cancer, penile cancer, pituitary tumor, prostate cancer, retinoblastoma, rhabdomyosarcoma, salivary gland cancer, sarcoma (adult soft tissue cancer), melanoma skin cancer, non-melanoma skin cancer, stomach cancer, testicular cancer, thymus cancer, thyroid cancer, uterine sarcoma, vaginal cancer, vulvar cancer, Waldenstrom's macroglobulinemia and cancers of viral origin. In some embodiments, the formulation is an oral formulation, such as a tablet or capsule. In some embodiments, the formulation is a parenteral formulation, such as in intravenous or intraperitoneal injection.

In some embodiments, the solubility of the BA is at least about 1.5 times the solubility of that compound in pure water. In some embodiments, the solubility of the compound is at least about 2 times the solubility of that compound in pure water. In some embodiments, the solubility of the compound is at least about 5 times the solubility of that compound in pure water. In some embodiments, the compound is at least about 10 times the solubility of that compound in pure water. In some embodiments, the compound is at least about 50 times the solubility of that compound in pure water.

### Definitions

"Nitrobenzamide compound(s)" means 4-iodo-3-nitrobenzamide ("Compound (Ia)" / "BA").

"Solubility" generally means the amount of a compound dissolved in a solvent. Suitable solvents include aqueous and non-aqueous solvents.

A compound is "dissolved" when it is "in solution", and does not spontaneously come out of solution to form a separate phase. In order to be dissolved, the compound need not dissociate completely on a molecular level, but must remain in solution so as to be effective in treatment of a disease or condition. A dissolved compound may be present in a micellar, emulsified, or liposomal form. A solution with dissolved compound will generally be clear.

"Poor solubility" means a small amount of compound dissolved in a solvent. Poor solubility is not an absolute term, but depends on the amount of the compound that is needed for effective treatment of a disease or condition. A compound will be poorly soluble if its solubility is lower than is desired in order for an effective treatment of a disease or condition.

"Enhanced solubility" means higher solubility than for the nitrobenzamide compound alone.

The term "treating" and its grammatical equivalents as used herein include attempting to achieve a therapeutic benefit and/or a prophylactic benefit. By therapeutic benefit is meant eradication or amelioration of the underlying disorder being treated. For example, in a cancer patient, therapeutic benefit includes eradication or amelioration of the underlying cancer. Also, a therapeutic benefit is achieved with the eradication or amelioration of one or more of the physiological symptoms associated with the underlying disorder such that an improvement is observed in the patient, notwithstanding the fact that the patient may still be afflicted with the underlying disorder. For prophylactic benefit, a composition of the invention may be administered to a patient at risk of developing cancer, or to a patient reporting one or more of the physiological symptoms of such conditions, even though a diagnosis of the condition may not have been made.

Enhanced solubility in water can be useful because many bodily fluids such as blood are water based (aqueous), and therefore, a more water soluble drug can have higher bioavailability. While the exact solubility of a compound in pure water is not the same as in an aqueous solution such as blood, a composition's solubility in pure water is often a good indication of solubility in other aqueous solutions.

The present invention provides pharmaceutical compositions containing nitrobenzamide compounds that have enhanced solubility in water.

In a preferred embodiment of the invention, the pharmaceutical composition of the invention will have a solubility of aromatic benzamide in aqueous solution greater than 1.5 times the solubility of the aromatic benzamide compound in pure water; preferably the solubility is greater than 2 times higher, more preferably the solubility is greater than 5 times higher, even more preferably the solubility is greater that 10 times higher, and most preferably the solubility is greater than 50 times higher.

While generally high drug solubility is desired, is would be appreciated by persons of ordinary skill in the art that there are other considerations in creating a pharmaceutical composition such as viscosity, stability, potential toxicity, etc. that may result a composition with lower solubility being more desirable for a particular therapy or delivery method as long as the amount of available drug is enough for the application. The present invention allows the pharmaceutical formulator the ability to optimize these factors.

The present invention provides for the use of pharmaceutical compositions containing nitrobenzamide compounds for the treatment of various cancers, including leukemia, breast cancer, ovarian cancer, lung cancer, bladder cancer, prostate cancer, pancreatic cancer, and cervical cancer, as well as other cancer types such as other breast cancers including a ductal carcinoma in a mammary gland, other forms of leukemia including acute promyleocytic leukemia in peripheral blood, ovarian cancer, lung cancer, bladder cancer, prostate cancer, pancreatic cancer, and cervical cancer, as well as other cancer types described herein.

It has been reported that nitrobenzamide compounds have selective cytotoxicity upon malignant cancer cells but not upon nonmalignant cancer cells. See Rice et at., Proc. Natl. Acad. Sci. USA 89:7703-7707 (1992). In one embodiment, the pharmaceutical compositions of the present invention may exhibit more selective toxicity towards tumor cells than non-tumor cells.

It has been reported that the tumorgenicity of nitrobenzamide and nitrososbenzamide compounds is enhanced when buthionine sulfoximine BSO is co-administered to cancer cells. See Mendeleyev et al., Biochemical Pharmacol. 50(5):705-714 (1995). Buthionine sulfoximine (BSO) inhibits gamma-glutamylcysteine sythetase, a key enzyme in the biosynthesis of glutathione, which is responsible in part for cellular resistance to chemotherapy. See Chen et al., Chem Biol Interact. Apr 24;111-112:263-75 (1998). The invention also provides pharmaceutical compounds useful for treating cancer via the administration of a nitrobenzamide compound in combination with BSO.

Cyclodextrins are cyclic carbohydrates that are derived from starch. The unmodified cyclodextrins differ by the number of glucopyranose units joined together in the cylindrical structure. The parent cyclodextrins contain 6, 7, or 8 glucopyranose units and are referred to as alpha.-, .beta.-, and .gamma.-cyclodextrin respectively. Each cyclodextrin subunit has secondary hydroxyl groups at the 2 and 3-positions and a primary hydroxyl group at the 6-position. The cyclodextrins may be pictured as hollow truncated cones with hydrophilic exterior surfaces and hydrophobic interior cavities. In aqueous solutions, these hydrophobic cavities provide a haven for hydrophobic organic compounds, which can fit all, or part of their structure into these cavities. This process, known as inclusion complexation, may result in increased apparent aqueous solubility and stability for the complexed drug.

Chemical modification of the parent cyclodextrins (usually at the hydroxyl moieties) has resulted in derivatives with sometimes improved safety while retaining or improving the complexation ability of the cyclodextrin. Some preferred derivatives of cyclodextrins are; the 2-hydroxypropyl derivatives, e.g. hydroxypropyl-β-cyclodextrin (Kleptose® from Roquette) (HPBCD) and the sulfoalkyl ether derivatives (SAE-CDs), e.g. sulfobutyl ether- β-cyclodextrin (Captisol® from Cydex) (SBEBCD).

The SAE-CDs are a class of negatively charged cyclodextrins, which vary in the nature of the alkyl spacer, the salt form, the degree of substitution and the starting parent cyclodextrin.

The anionic sulfobutyl ether substituent dramatically improves the aqueous solubility of the parent cyclodextrin. Reversible, non-covalent, complexation of drugs with the Captisol® cyclodextrin generally allows for increased solubility and stability of drugs in aqueous solutions.

The preferred weight ratio of cyclodextrin to compound (Ia) is from 1:100 to 5,000:1. A preferred formulation is 1 g BA to 25 g cyclodextrin. About 25% cyclodextrin is most preferred. Also, 40% cyclodextrin is suitable.

Surfactants are compounds that have surface active properties. Surfactants are amphiphilic molecules which are manufactured by chemical processes or purified from natural sources or processes. These can be anionic, cationic, nonionic, and zwitterionic. Typical surfactants are described in Emulsions: Theory and Practice, Paul Becher, Robert E. Krieger Publishing, Malabar, Fla., 1965; Pharmaceutical Dosage Forms: Dispersed Systems Vol. I, Martin M. Rigear, Surfactants and U.S. Pat. No. 5,595,723.

The pharmaceutical compositions of the present invention comprising surfactants can result in emulsions, suspensions, or other preparations, for example, liposomal preparations, may be used. With respect to liposomal preparations, any known methods for preparing liposomes for treatment of a condition may be used. See, for example, Bangham et al., J. Mol. Biol, 23: 238-252 (1965) and Szoka et al., Proc. Natl Acad. Sci 75: 4194-4198 (1978). Ligands may also be attached to the liposomes to direct these compositions to particular sites of action. The surfactants of the present invention can also be a wetting agent (e.g., lecithin, lysolecithin and/or a long-chain fatty alcohol).

One group of preferred surfactants for the present invention are non-ionic surfactants. Examples of useful non-ionic surfactants are Poloxamers or pluronics, which are synthetic block copolymers of ethylene oxide and propylene oxide having the general structure:

H(OCH₂CH₂)ₐ (OCH₂CH₂CH₂)_{b}(OCH₂CH₂)ₐOH.

The following variants based on the values of a and b are commercially available from BASF Performance Chemicals (Parsippany, N.J.) under the trade name Pluronic and which consist of the group of surfactants designated by the CTFA (Cosmetic, toiletry, and fragrance association) name of Poloxamer 108, 188, 217, 237, 238, 288, 338, 407, 101, 105, 122, 123, 124, 181, 182, 183, 184, 212, 231, 282, 331, 401, 402, 185, 215, 234, 235, 284, 333, 334, 335, and 403. For the most commonly used poloxamers 124, 188, 237, 338 and 407 the values of a and b are 12/20, 79/28, 64/37, 141/44 and 101/56, respectively.

The pharmaceutical compositions of the present invention may also comprise the surfactant Solutol HS-15 which is a polyethylene glycol 660 hydroxystearate manufactured by BASF.

The pharmaceutical compositions of the present invention also comprise surfactants selected from a group of non-ionic surfactants including, without limitation thereto, polyoxyethylene sorbitan fatty acid esters such as Polysorbates 20, 60 and 80; polyoxyethylene alkyl ethers such as Brij's (e.g., BRIJ 97 or BRIJ 98 from ICI Surfactants, Cremophors (such as Cremophor RH or Cremophor EL), Volpo (e.g., VOLPO 10 and VOLPO 20 from Croda, Inc.) and equivalents thereof-.

Hydrophile-lipophile balance: An empirical formula used to index surfactants. Its value varies from 1-45 and in the case of non-ionic surfactants from about 1-20. In general for lipophilic surfactants the HLB is less than 10 and for hydrophilic ones the HLB is greater than 10. Suitable formulations include Polysorbate 80 and 20 at 100% and 10%, cremophor solutions at 10%, and Solutol at 25% and 30%.

Preferred surfactants for the present invention are polyethylene sorbitan monooleate (Polysorbate 80), polyoxyethylene [20] sorbitan monolaurate (Polysorbate 20), Cremophor EL (BASF), Cremophor RH40 (BASF), Poloxamer 118, and Solutol HS-15 (BASF).

The pharmaceutical compositions of the present invention may also comprise co-solvents. Co-solvents are at least partially miscible with water and can result in increased solubility of the nitrobenzamide compound.

In a preferred embodiment, the co-solvent comprises ethanol, glycofurol, glycerin formal, benzyl alcohol, PEG 400, propylene glycol, or N, N-dimethyl acetamide (DMA).

The preferred weight ratio of co-solvent to compound (Ia) in the pharmaceutical composition is from 1:100 to 10,000:1. Most preferred range is about 1:50 to about 1:250.

It is desirable that the pharmaceutical compositions of the invention are biocompatible, meaning that they are capable of performing functions within or upon a living organism in an acceptable manner, without undue toxicity or physiological or pharmacological effects.

The pharmaceutical compositions of the present may comprise mixtures of solubilizers including combinations of cyclodextrins, surfactants, and/or co-solvents. These mixtures can comprise cyclodextrins and surfactants, cyclodextrins and co-solvents, and mixtures of cyclodextrias, surfactants, and co-solvents. The compositions can also comprise mixtures of each of the types of solubilizer such as more than one type of cyclodextrin, more than one type of surfactant, and/or more than one type of co-solvent.

### Formulations, Routes of Administration, and Effective Doses

Another aspect of the invention is the use of the pharmaceutical compositions as an anti-viral or an anti-tumor treatment. The pharmaceutical compositions of the present invention may be provided as a prodrug and/or may be allowed to interconvert to a nitrosobenzamide form in vivo after administration. That is, either the nitrobenzamide form and/or the nitrosobenzamide form, or pharmaceutically acceptable salts may be used in developing a formulation for use in the present invention. Further, in some embodiments, the compound may be used in combination with one or more other compounds or in one or more other forms. For example a formulation may comprise both the nitrobenzamide compound and acid forms in particular proportions, depending on the relative potencies of each and the intended indication.The pharmaceutical compositions of the present invention can be combined with other active ingredients.

The term "pharmaceutically acceptable salt" means those salts which retain the biological effectiveness and properties of the compounds used in the present invention, and which are not biologically or otherwise undesirable. For example, a pharmaceutically acceptable salt does not interfere with the beneficial effect of the compound of the invention in treating a cancer or a virus.

Typical salts are those of the inorganic ions, such as, for example, sodium, potassium, calcium and magnesium ions. Such salts include salts with inorganic or organic acids, such as hydrochloric acid, hydrobromic acid, phosphoric acid, nitric acid, sulfuric acid, methanesulfonic acid, p-toluenesulfonic acid, acetic acid, fumaric acid, succinic acid, lactic acid, mandelic acid, malic acid, citric acid, tartaric acid or maleic acid. In addition, if the compounds used in the present invention contain a carboxy group or other acidic group, it may be converted into a pharmaceutically acceptable addition salt with inorganic or organic bases. Examples of suitable bases include sodium hydroxide, potassium hydroxide, ammonia, cyclohexylamine, dicyclohexyl-amine, ethanolamine, diethanolamine and triethanolamine.

For injection, the inhibitors of the present invention are formulated in aqueous solutions, with physiologically compatible buffers such as Hank's solution, Ringer's solution, or physiological saline buffer. Such compositions may also include one or more excipients, for example, preservatives, solubilizers, tillers, lubricants, stabilizers, albumin, and the like. Methods of formulation are known in the art, for example, as disclosed in Remington's Pharmaceutical Sciences, latest edition, Mack Publishing Co., Easton, PA.

Pharmaceutical compositions suitable for use in the present invention include compositions wherein the active ingredients are present in an effective amount, i.e., in an amount effective to achieve therapeutic and/or prophylactic benefit in at least one of the cancers described herein. The actual amount effective for a particular application will depend on the condition or conditions being treated, the condition of the subject, the formulation, and the route of administration, as well as other factors known to those of skill in the art. Determination of an effective amount of a nitrobenzamide compound is well within the capabilities of those skilled in the art, in light of the disclosure herein, and will be determined using routine optimization techniques.

### THERAPEUTIC USE OF PARP INHIBITORS

### Cancer types

The invention is useful for the treatment of several specific cancers or tumors. For example, cancer types include adrenal cortical cancer, anal cancer, aplastic anemia, bile duct cancer, bladder cancer, bone cancer, bone metastasis, Adult CNS brain tumors, Children CNS brain tumors, breast cancer, Castleman Disease, cervical cancer, Childhood Non-Hodgkin's lymphoma, colon and rectum (colorectal) cancer, endometrial cancer, esophagus cancer, Ewing's family of tumors, eye cancer, gallbladder cancer, gastrointestinal carcinoid tumors, gastrointestinal stromal tumors, gestational trophoblastic disease, Hodgkin's disease, Kaposi's sarcoma, kidney cancer, laryngeal and hypopharyngeal cancer, acute lymphocytic leukemia, acute myeloid leukemia, children's leukemia, chronic lymphocytic leukemia, chronic myeloid leukemia, liver cancer, lung cancer, lung carcinoid tumors, Non-Hodgkin's lymphoma, male breast cancer, malignant mesothelioma, multiple myeloma, myelodysplastic syndrome, nasal cavity and paranasal cancer, nasopharyngeal cancer, neuroblastoma, oral cavity and oropharyngeal cancer, osteosarcoma, ovarian cancer, pancreatic cancer, penile cancer, pituitary tumor, prostate cancer, retinoblastoma, rhabdomyosarcoma, salivary gland cancer, sarcoma (adult soft tissue cancer), melanoma skin cancer, non-melanoma skin cancer, stomach cancer, testicular cancer, thymus cancer, thyroid cancer, uterine sacrcoma, vaginal cancer, vulvar cancer, Waldenstrom's macroglobulinemia, cancers of viral origin and virus-associated cancers.

Carcinoma of the thyroid gland is the most common malignancy of the endocrine system. Carcinoma of the thyroid gland include differentiated tumors (papillary or follicular) and poorly differentiated tumors (medullary or anaplastic). Carcinomas of the vagina include squamous cell carcinoma, adenocarcinoma, melanoma and sarcoma. Testicular cancer is broadly divided into seminoma and nonseminoma types.

Thymomas are epithelial tumors of the thymus, which may or may not be extensively infiltrated by nonneoplastic lymphocytes. The term thymoma is customarily used to describe neoplasms that show no overt atypia of the epithelial component. A thymic epithelial tumor that exhibits clear-cut cytologic atypia and histologic features no longer specific to the thymus is known as a thymic carcinoma (also known as type C thymoma).

The compositions provided by the invention may be administered in combination with other therapies. The choice of therapy that can be co-administered with the compositions of the invention will depend, in part, on the condition being treated. For example, for treating acute myleoid leukemia, the benzamide compound can be used in combination with radiation therapy, monoclonal antibody therapy, chemotherapy, bone marrow transplantation, gene therapy, immunotherapy, or a combination thereof.

### Breast Cancer

In one aspect, the invention is used for treating breast cancer, preferably a ductal carcinoma in duct tissue in a mammary gland.

Several types of breast cancer exist that may be treated by the compositions provided by the invention. A lobular carcinoma in situ and a ductal carcinoma in situ are breast cancers that have developed in the lobules and ducts, respectively, but have not spread to the fatty tissue surrounding the breast or to other areas of the body. An infiltrating (or invasive) lobular and a ductal carcinoma are cancers that have developed in the lobules and ducts, respectively, and have spread to either the breast's fatty tissue and/or other parts of the body. Other cancers of the breast that would benefit from treatment by the compositions provided by the invention are medullary carcinomas, colloid carcinomas, tubular carcinomas, and inflammatory breast cancer.

Treatments available for breast cancer patients are surgery, immunotherapy, radiation therapy, chemotherapy, endocrine therapy, or a combination thereof. A lumpectomy and a mastectomy are two possible surgical procedures available for breast cancer patients.

Chemotherapy utilizes anti-tumor agents to prevent cancer cells from multiplying, invading, metastasizing and killing a patient. Several drugs are available to treat breast cancer, including cytotoxic drugs such as doxorubicin, cyclophosphamide, methotrexate, paclitaxel, thiotepa, mitoxantrone, vincristine, or combinations thereof. Endocrine therapy may be an effective treatment where the remaining breast tissue retains endocrine sensitivity. Agents administered for this therapy include tamoxifen, megestrol acetate, aminoglutethimide, fluoxymesterone, leuprolide, goserelin, and prednisone.

The compositions provided by the invention can provide a beneficial effect for breast cancer patients, by administration of the nitrobenzamide compound or a combination of administration of the nitrobenzamide compound and surgery, radiation therapy, chemotherapy, or endocrine therapy.

### Ovarian cancer

In another aspect, the invention is used for treating ovarian cancer, including epithelial ovarian tumors. Preferably, the invention provides a composition for treating an ovarian cancer selected from the following: an adenocarcinoma in the ovary and an adenocarcinoma that has migrated from the ovary into the abdominal cavity. Surgery, immunotherapy, chemotherapy, hormone therapy, radiation therapy, or a combination thereof are some possible treatments available for ovarian cancer. Some possible surgical procedures include debulking, and a unilateral or bilateral oophorectomy and/or a unilateral or bilateral salpigectomy.

Anti-cancer drugs that may be used include cyclophosphamide, etoposide, altretamine, and ifosfamide. Hormone therapy with the drug tamoxifen may be used to shrink ovarian tumors. Radiation therapy may be external beam radiation therapy and/or brachytherapy.

The compositions provided by the invention can provide a beneficial effect for ovarian cancer patients, by administration of the nitrobenzamide compound or a combination of administration of the nitrobenzamide compound and surgery, radiation therapy, chemotherapy endocrine therapy, or a combination thereof.

### Cervical Cancer

In another aspect, the invention is used for treating cervical cancer, preferably an adenocarcinoma in the cervix epithelial. Two main types of this cancer exist: squamous cell carcinoma and adenocarcinomas. The former constitutes about 80-90% of all cervical cancers and develops where the ectocervix (portion closest to the vagina) and the endocervix (portion closest to the uterus) join. The latter develop in the mucous-producing gland cells of the endocervix. Some cervical cancers have characteristics of both of these and are called adenosquamous carcinomas or mixed carcinomas.

The chief treatments available for cervical cancer are surgery, immunotherapy, radiation therapy and chemotherapy. Some possible surgical options are cryosurgery, a hysterectomy, and a radical hysterectomy. Radiation therapy for cervical cancer patients includes external beam radiation therapy or brachytherapy. Anti-cancer drugs that may be administered as part of chemotherapy to treat cervical cancer include cisplatin, carboplatin, hydroxyurea, irinotecan, bleomycin, vincrinstine, mitomycin, ifosfamide, fluorouracil, etoposide, methotrexate, and combinations thereof.

The compositions provided by the invention can provide a beneficial effect for cervical cancer patients, by administration of the nitrobenzamide compound or a combination of administration of the nitrobenzamide compound and surgery, radiation therapy, chemotherapy, or a combination thereof.

### Prostate Cancer

In one other aspect, the invention is used to treat prostate cancer, preferably a prostate cancer selected from the following: an adenocarcinoma or an adenocarinoma that has migrated to the bone. Prostate cancer develops in the prostate organ in men, which surrounds the first part of the urethra. The prostate has several cell types but 99% of tumors are adenocarcinomas that develop in the glandular cells responsible for generating seminal fluid.

Surgery, immunotherapy, radiation therapy, cryosurgery, hormone therapy, and chemotherapy are some treatments available for prostate cancer patients. Possible surgical procedures to treat prostate cancer include radical retropubic prostatectomy, a radical perineal prostatectomy, and a laparoscopic radical prostatectomy. Some radiation therapy options are external beam radiation, including three dimensional conformal radiation therapy, intensity modulated radiation therapy, and conformal proton beam radiation therapy. Brachytherapy (seed implantation or interstitial radiation therapy) is also an available method of treatment for prostate cancer. Cryosurgery is another possible method used to treat localized prostate cancer cells.

Hormone therapy, also called androgen deprivation therapy or androgen suppression therapy, may be used to treat prostate cancer. Several methods of this therapy are available including an orchiectomy in which the testicles, where 90% of androgens are produced, are removed. Another method is the administration of luteinizing hormone-releasing hormone (LHRH) analogs to lower androgen levels. The LHRH analogs available include leuprolide, goserelin, triptorelin, and histrelin. An LHRH antagonist may also be administered, such as abarelix.

Treatment with an anti-androgen agent, which blocks androgen activity in the body, is another available therapy. Such agents include flutamide, bicalutamide, and nilutamide. This therapy is typically combined with LHRH analog administration or an orchiectomy, which is termed a combined androgen blockade (CAB).

Chemotherapy may be appropriate where a prostate tumor has spread outside the prostate gland and hormone treatment is not effective. Anti-cancer drugs such as doxorubicin, estramustine, etoposide, mitoxantrone, vinblastine, paclitaxel, docetaxel, carboplatin, and prednisone may be administered to slow the growth of prostate cancer, reduce symptoms and improve the quality of life.

The compositions provided by the invention can provide a beneficial effect for prostate cancer patients, by administration of the nitrobenzamide compound or a combination of administration of the nitrobenzamide compound and surgery, radiation therapy, chemotherapy, hormone therapy, or a combination thereof.

### Pancreatic Cancer

Some embodiments are used for treating pancreatic cancer, preferably a pancreatic cancer selected from the following: an epitheliod carcinoma in the pancreatic duct tissue and an adenocarcinoma in a pancreatic duct.

The most common type of pancreatic cancer is an adenocarcinoma, which occurs in the lining of the pancreatic duct. The possible treatments available for pancreatic cancer are surgery, immunotherapy, radiation therapy, and chemotherapy. Possible surgical treatment options include a distal or total pancreatectomy and a pancreaticoduodenectomy (Whipple procedure).

Radiation therapy may be an option for pancreatic cancer patients, specifically external beam radiation where radiation is focused on the tumor by a machine outside the body. Another option is intraoperative electron beam radiation administered during an operation.

Chemotherapy may be used to treat pancreatic cancer patients. Appropriate anti-cancer drugs include 5-fluorouracil (5-FU), mitomycin, ifosfamide, doxorubicin, streptozocin, chlorozotocin, and combinations thereof.

The compositions provided by the invention can provide a beneficial effect for pancreatic cancer patients, by administration of the nitrobenzamide compound or a combination of administration of the nitrobenzamide compound and surgery, radiation therapy, or chemotherapy.

### Bladder Cancer

Some embodiments are used for treating bladder cancer, preferably a transitional cell carcinoma in urinary bladder. Bladder cancers are urothelial carcinomas (transitional cell carcinomas) or tumors in the urothelial cells that line the bladder. The remaining cases of bladder cancer are squamous cell carcinomas, adenocarcinomas, and small cell cancers. Several subtypes of urothelial carcinomas exist depending on whether they are noninvasive or invasive and whether they are papillary, or flat. Noninvasive tumors are in the urothelium, the innermost layer of the bladder, while invasive tumors have spread from the urothelium to deeper layers of the bladder's main muscle wall. Invasive papillary urothelial carcinomas are slender finger-like projections that branch into the hollow center of the bladder and also grow outward into the bladder wall. Non-invasive papillary urothelial tumors grow towards the center of the bladder. While a non-invasive, flat urothelial tumor (also called a flat carcinoma in situ) is confined to the layer of cells closest to the inside hollow part of the bladder, an invasive flat urothelial carcinoma invades the deeper layer of the bladder, particularly the muscle layer.

To treat bladder cancer, surgery, radiation therapy, immunotherapy, chemotherapy, or a combination thereof may be applied. Some possible surgical options are a transurethral resection, a cystectomy, or a radical cystectomy. Radiation therapy for bladder cancer may include external beam radiation and brachytherapy.

Immunotherapy is another method that may be used to treat a bladder cancer patient. Typically this is accomplished intravesically, which is the administration of a treatment agent directly into the bladder by way of a catheter. One method is Bacillus Calmete-Guerin (BCG) where a bacterium sometimes used in tuberculosis vaccination is given directly to the bladder through a catheter. The body mounts an immune response to the bacterium, thereby attacking and killing the cancer cells.

Another method of immunotherapy is the administration of interferons, glycoproteins that modulate the immune response. Interferon alpha is often used to treat bladder cancer.

Anti-cancer drugs that may be used in chemotherapy to treat bladder cancer include thitepa, methotrexate, vinblastine, doxorubicin, cyclophosphamide, paclitaxel, carboplatin, cisplatin, ifosfamide, gemcitabine, or combinations thereof.

The compositions provided by the invention can provide a beneficial effect for bladder cancer patients, by administration of the nitrobenzamide compound or a combination of administration of the nitrobenzamide compound and surgery, radiation therapy, immunotherapy, chemotherapy, or a combination thereof.

### Acute Myeloid Leukemia

Some embodiments are used for treating acute myeloid leukemia (AML), preferably acute promyleocytic leukemia in peripheral blood. AML begins in the bone marrow but can spread to other parts of the body including the lymph nodes, liver, spleen, central nervous system, and testes. It is acute meaning it develops quickly and may be fatal if not treated within a few months. AML is characterized by immature bone marrow cells usually granulocytes or monocytes, which continue to reproduce and accumulate.

AML may be treated by immunotherapy, radiation therapy, chemotherapy, bone marrow or peripheral blood stem cell transplantation, or a combination thereof. Radiation therapy includes external beam radiation and may have side effects. Anti-cancer drugs that may be used in chemotherapy to treat AML include cytarabine, anthracycline, anthracenedione, idarubicin, daunorubicin, idarubicin, mitoxantrone, thioguanine, vincristine, prednisone, etoposide, or a combination thereof.

Monoclonal antibody therapy may be used to treat AML patients. Small molecules or radioactive chemicals may be attached to these antibodies before administration to a patient in order to provide a means of killing leukemia cells in the body. The monoclonal antibody, gemtuzumab ozogamicin, which binds CD33 on AML cells, may be used to treat AML patients unable to tolerate prior chemotherapy regimens.

Bone marrow or peripheral blood stem cell transplantation may be used to treat AML patients. Some possible transplantation procedures are an allogenic or an autologous transplant.

The compositions provided by the invention can provide a beneficial effect for leukemia patients, by administration of the nitrobenzamide compound or a combination of administration of the nitrobenzamide compound and surgery, radiation therapy, chemotherapy, or transplantation therapy.

There are other types of leukemias that can also be treated by the compositions provided by the invention including but not limited to, Acute Lymphocytic Leukemia, Acute Myeloid Leukemia, Chronic Lymphocytic Leukemia, Chronic Myeloid Leukemia, Hairy Cell Leukemia, Myelodysplasia, and Myeloproliferative Disorders.

### Lung Cancer

Some embodiments are used to treat lung cancer. The most common type of lung cancer is non-small cell lung cancer (NSCLC), which accounts for approximately 80-85% of lung cancers and is divided into squamous cell carcinomas, adenocarcinomas, and large cell undifferentiated carcinomas. Small cell lung cancer accounts for 15-20% of lung cancers.

Treatment options for lung cancer include surgery, immunotherapy, radiation therapy, chemotherapy, photodynamic therapy, or a combination thereof. Some possible surgical options for treatment of lung cancer are a segmental or wedge resection, a lobectomy, or a pneumonectomy. Radiation therapy may be external beam radiation therapy or brachytherapy.

Some anti-cancer drugs that may be used in chemotherapy to treat lung cancer include cisplatin, carboplatin, paclitaxel, docetaxel, gemcitabine, vinorelbine, irinotecan, etoposide, vinblastine, gefitinib, ifosfamide, methotrexate, or a combination thereof. Photodynamic therapy (PDT) may be used to treat lung cancer patients.

The compositions described herein can provide a beneficial effect for lung cancer patients, by administration of the nitrobenzamide compound or a combination of administration of the nitrobenzamide compound and surgery, radiation therapy, chemotherapy, photodynamic therapy, or a combination thereof.

### Skin Cancer

Some embodiments are used for treating skin cancer. There are several types of cancer that start in the skin. The most common types are basal cell carcinoma and squamous cell carcinoma, which are non-melanoma skin cancers. Actinic keratosis is a skin condition that sometimes develops into squamous cell carcinoma. Non-melanoma skin cancers rarely spread to other parts of the body. Melanoma, the rarest form of skin cancer, is more likely to invade nearby tissues and spread to other parts of the body. Different types of treatment are available for patients with non-melanoma and melanoma skin cancer and actinic keratosis including surgery, radiation therapy, chemotherapy and photodynamic therapy. Some possible surgical options for treatment of skin cancer are mohs micrographic surgery, simple excision, electrodesiccation and curettage, cryosurgery, laser surgery. Radiation therapy may be external beam radiation therapy or brachytherapy. Other types of treatments that are being tested in clinical trials are biologic therapy or immunotherapy, chemoimmunotherapy, topical chemotherapy with fluorouracil and photodynamic therapy.

The compositions provided by the invention can provide a beneficial effect for skin cancer patients, by administration of the nitrobenzamide compound or a combination of administration of the nitrobenzamide compound and surgery, radiation therapy, chemotherapy, photodynamic therapy, or a combination thereof.

### Eye Cancer, Retinoblastoma

Some embodiments are used to treat eye retinoblastoma. Retinoblastoma is a malignant tumor of the retina. Although retinoblastoma may occur at any age, it most often occurs in younger children, usually before the age of 5 years. The tumor may be in one eye only or in both eyes. Retinoblastoma is usually confined to the eye and does not spread to nearby tissue or other parts of the body. Treatment options that attempt to cure the patient and preserve vision include enucleation (surgery to remove the eye), radiation therapy, cryotherapy, photocoagulation, immunotherapy, thermotherapy and chemotherapy. Radiation therapy may be external beam radiation therapy or brachytherapy.

The compositions provided by the invention can provide a beneficial effect for eye retinoblastoma patients, by administration of the nitrobenzamide compound or a combination of administration of the nitrobenzamide compound and surgery, radiation therapy, cryotherapy, photocoagulation, thermotherapy and chemotherapy, or a combination thereof.

### Eye Cancer, Intraocular Melanoma

Some embodiments are used to treat intraocular (eye) melanoma. Intraocular melanoma, a rare cancer, is a disease in which cancer cells are found in the part of the eye called the uvea. The uvea includes the iris, the ciliary body, and the choroid. Intraocular melanoma occurs most often in people who are middle aged. Treatments for intraocular melanoma include surgery, immunotherapy, radiation therapy and laser therapy. Surgery is the most common treatment of intraocular melanoma. Some possible surgical options are iridectomy, iridotrabeculectomy, iridocyclectomy, choroidectomy, enucleation and orbital exenteration. Radiation therapy may be external beam radiation therapy or brachytherapy. Laser therapy may be an intensely powerful beam of light to destroy the tlimor, thermotherapy or photocoagulation.

The compositions provided by the invention can provide a beneficial effect for intraocular melanoma patients, by administration of the nitrobenzamide compound or a combination of administration of the nitrobenzamide compound and surgery, radiation therapy and laser therapy, or a combination thereof.

### Endometrium Cancer

Some embodiments are used for treating endometrium cancer. Endometrial cancer is a cancer that starts in the endometrium, the inner lining of the uterus. Some of the examples of the cancer of uterus and endometrium include, but are not limited to, adenocarcinomas, adenoacanthomas, adenosquamous carcinomas, papillary serous adenocarcinomas, clear cell adenocarcinomas, uterine sarcomas, stromal sarcomas, malignant mixed mesodermal tumors, and leiomyosarcomas.

The compositions provided by the invention can provide a beneficial effect for endometrium cancer patients, by administration of the nitrobenzamide compound or a combination of administration of the nitrobenzamide compound and surgery, radiation therapy, chemotherapy, gene therapy, photodynamic therapy, antiangiogenesis therapy, and immunotherapy, or a combination thereof.

### Liver Cancer

Some embodimentsare used to treat primary liver cancer (cancer that begins in the liver). Primary liver cancer can occur in both adults and children. Different types of treatments are available for patients with primary liver cancer. These include surgery, immunotherapy, radiation therapy, chemotherapy and percutaneous ethanol injection. The types of surgery that may be used are cryosurgery, partial hepatectomy, total hepatectomy and radiofrequency ablation. Radiation therapy may be external beam radiation therapy, brachytherapy, radiosensitizers or radiolabel antibodies. Other types of treatment include hyperthermia therapy and immunotherapy.

The compositions provided by the invention can provide a beneficial effect for liver cancer patients, by administration of the nitrobenzamide compound or a combination of administration of the nitrobenzamide compound and surgery, radiation therapy, chemotherapy, percutaneous ethanol injection, hyperthermia therapy and immunotherapy, or a combination thereof.

### Kidney Cancer

Some embodiments are used to treat kidney cancer. Kidney cancer (also called renal cell cancer or renal adenocarcinoma) is a disease in which malignant cells are found in the lining of tubules in the kidney. Kidney cancer may be treated by surgery, radiation therapy, chemotherapy and immunotherapy. Some possible surgical options to treat kidney cancer are partial nephrectomy, simple nephrectomy and radical nephrectomy. Radiation therapy may be external beam radiation therapy or brachytherapy. Stem cell transplant may be used to treat kidney cancer.

The compositions provided by the invention can provide a beneficial effect for kidney cancer patients, by administration of the nitrobenzamide compound or a combination of administration of the nitrobenzamide compound and surgery, radiation therapy, chemotherapy, and immunotherapy, or a combination thereof.

### Thyroid Cancer

Some embodiments are used for treating thyroid cancer. Thyroid cancer is a disease in which cancer (malignant) cells are found in the tissues of the thyroid gland. The four main types of thyroid cancer are papillary, follicular, medullary and anaplastic. Thyroid cancer may be treated by surgery, immunotherapy, radiation therapy, hormone therapy and chemotherapy. Surgery is the most common treatment of thyroid cancer. Some possible surgical options for treatment of thyroid cancer are lobectomy, near-total thyroidectomy, total thyroidectomy and lymph node dissection. Radiation therapy may be external radiation therapy or may required intake of a a liquid that contains radioactive iodine. Hormone therapy uses hormones to stop cancer cells from growing. In treating thyroid cancer, hormones can be used to stop the body from making other hormones that might make cancer cells grow.

The compositions provided by the invention can provide a beneficial effect for thyroid cancer patients, by administration of the nitrobenzamide compound or a combination of administration of the nitrobenzamide compound and surgery, surgery, radiation therapy, hormone therapy and chemotherapy, or a combination thereof.

### AIDS Related Cancers

### AIDS-Related Lymphoma

Some embodiments are used for treating AIDS-related lymphoma. AIDS-related lymphoma is a disease in which malignant cells form in the lymph system of patients who have acquired immunodeficiency syndrome (AIDS). AIDS is caused by the human immunodeficiency virus (HIV), which attacks and weakens the body's immune system. The immune system is then unable to fight infection and diseases that invade the body. People with HIV disease have an increased risk of developing infections, lymphoma, and other types of cancer. Lymphomas are cancers that affect the white blood cells of the lymph system. Lymphomas are divided into two general types: Hodgkin's lymphoma and non-Hodgkin's lymphoma. Both Hodgkin's lymphoma and non-Hodgkin's lymphoma may occur in AIDS patients, but non-Hodgkin's lymphoma is more common. When a person with AIDS has non-Hodgkin's lymphoma, it is called an AIDS-related lymphoma. Non-Hodgkin's lymphomas may be indolent (slow-growing) or aggressive (fast-growing). AIDS-related lymphoma is usually aggressive. The three main types of AIDS-related lymphoma are diffuse large B-cell lymphoma, B-cell immunoblastic lymphoma and small non-cleaved cell lymphoma.

Treatment of AIDS-related lymphoma combines treatment of the lymphoma with treatment for AIDS. Patients with AIDS have weakened immune systems and treatment can cause further damage. For this reason, patients who have AIDS-related lymphoma are usually treated with lower doses of drugs than lymphoma patients who do not have AIDS. Highly-active antiretroviral therapy (HAART) is used to slow progression of HIV. Medicine to prevent and treat infections, which can be serious, is also used. AIDS-related lymphomas may be treated by chemotherapy, immunotherapy, radiation therapy and high-dose chemotherapy with stem cell transplant. Radiation therapy may be external beam radiation therapy or brachytherapy. AIDS-related lymphomas can be treated by monoclonal antibody therapy.

The compositions provided by the invention can provide a beneficial effect for AIDS-related lymphoma patients, by administration of the nitrobenzamide compound or a combination of administration of the nitrobenzamide compound and chemotherapy, radiation therapy and high-dose chemotherapy, or a combination thereof.

### Kaposi's Sarcoma

Some embodiments are used for treating Kaposi's sarcoma. Kaposi's sarcoma is a disease in which cancer cells are found in the tissues under the skin or mucous membranes that line the mouth, nose, and anus. Classic Kaposi's sarcoma usually occurs in older men of Jewish, Italian, or Mediterranean heritage. This type of Kaposi's sarcoma progresses slowly, sometimes over 10 to 15 years. Kaposi's sarcoma may occur in people who are taking immunosuppressants. Kaposi's sarcoma in patients who have Acquired Immunodeficiency Syndrome (AIDS) is called epidemic Kaposi's sarcoma. Kaposi's sarcoma in people with AIDS usually spreads more quickly than other kinds of Kaposi's sarcoma and often is found in many parts of the body. Kaposi's sarcoma may be treated with surgery, chemotherapy, radiation therapy and immunotherapy. External radiation therapy is a common treatment of Kaposi's sarcoma. Some possible surgical options to treat Kaposi's Sarcoma are local excision, electrodessication and curettage, and cryotherapy.

The compositions provided by the invention can provide a beneficial effect for Kaposi's sarcoma, by administration of the nitrobenzamide compound or a combination of administration of the nitrobenzamide compound and surgery, chemotherapy, radiation therapy and immunotherapy, or a combination thereof.

### Viral-Induced Cancers

Some embodiments are used for treating viral-induced cancers. Several common viruses are clearly or probable causal factors in the etiology of specific malignancies. These viruses either normally establish latency or few can become persistent infections. Oncogenesis is probably linked to an enhanced level of viral activation in the infected host, reflecting heavy viral dose or compromised immune control. The major virus-malignancy systems include hepatitis B virus (HBV), hepatitis C virus (HCV), and hepatocellular carcinoma; human lymphotropic virus-type 1 (HTLV-1) and adult T-cell leukemia/lymphoma; and human papilloma virus (HPV) and cervical cancer. In general, these malignancies occur relatively early in life, typically peaking in middle-age or earlier.

### Virus-Induced Hepatocellular Carcinoma

The causal relationship between both HBV and HCV and hepatocellular carcinoma or liver cancer is established through substantial epidemiologic evidence. Both appear to act via chronic replication in the liver by causing cell death and subsequent regeneration. Different types of treatments are available for patients with liver cancer. These include surgery, immunotherapy, radiation therapy, chemotherapy and percutaneous ethanol injection. The types of surgery that may be used are cryosurgery, partial hepatectomy, total hepatectomy and radiofrequency ablation. Radiation therapy may be external beam radiation therapy, brachytherapy, radiosensitizers or radiolabel antibodies. Other types of treatment include hyperthermia therapy and immunotherapy.

The compositions provided by the invention can provide a beneficial effect for virus induce hepatocellular carcinoma patients, by administration of the nitrobenzamide compound or a combination of administration of the nitrobenzamide compound and surgery, radiation therapy, chemotherapy, percutaneous ethanol injection, hyperthermia therapy and immunotherapy, or a combination thereof.

### Viral-Induced Adult T cell leukemia/lymphoma

The association between HTLV-1 and Adult T cell leukemia (ATL) is firmly established. Unlike the other oncogenic viruses found throughout the world, HTLV-1 is highly geographically restricted, being found primarily in southern Japan, the Caribbean, west and central Africa, and the South Pacific islands. Evidence for causality includes the monoclonal integration of viral genome in almost all cases of ATL in carriers. The risk factors for HTLV- I -associated malignancy appear to be perinatal infection, high viral load, and being male sex.

Adult T cell leukemia is a cancer of the blood and bone marrow. The standard treatments for adult T cell leukemia/lymphoma are radiation therapy, immunotherapy, and chemotherapy. Radiation therapy may be external beam radiation therapy or brachytherapy. Other methods of treating adult T cell leukemia/lymphoma include immunotherapy and high-dose chemotherapy with stem cell transplantation.

The compositions provided by the invention can provide a beneficial affect for Adult T cell leukemia patients, by administration of the nitrobenzamide compound or a combination of administration of the nitrobenzamide compound and radiation therapy, chemotherapy, and immunotherapy, or a combination thereof.

### Viral-Induced Cervical Cancer

Infection of the cervix with human papillomavirus (HPV) is the most common cause of cervical cancer. Not all women with HPV infection, however, will develop cervical cancer. Cervical cancer usually develops slowly over time. Before cancer appears in the cervix, the cells of the cervix go through changes known as dysplasia, in which cells that are not normal begin to appear in the cervical tissue. Later, cancer cells start to grow and spread more deeply into the cervix and to surrounding areas. The standard treatments for cervical cancers are surgery, immunotherapy, radiation therapy and chemotherapy. The types of surgery that may be used are conization, total hysterectomy, bilateral salpingo-oophorectomy, radical hysterectomy, pelvic exenteration, cryosurgery, laser surgery and loop electrosurgical excision procedure. Radiation therapy may be external beam radiation therapy or brachytherapy.

The compositions provided by the invention can provide a beneficial effect for adult cervical cancer, by administration of the nitrobenzamide compound or a combination of administration of the nitrobenzamide compound and radiation therapy, chemotherapy, or a combination thereof.

### CNS cancers

Brain and spinal cord tumors are abnormal growths of tissue found inside the skull or the bony spinal column, which are the primary components of the central nervous system (CNS). Benign tumors are non-cancerous, and malignant tumors are cancerous. The CNS is housed within rigid, bony quarters (i.e., the skull and spinal column), so any abnormal growth, whether benign or malignant, can place pressure on sensitive tissues and impair function. Tumors that originate in the brain or spinal cord are called primary tumors. Most primary tumors are caused by out-of-control growth among cells that surround and support neurons. In a small number of individuals, primary tumors may result from specific genetic disease (e.g., neurofibromatosis, tuberous sclerosis) or from exposure to radiation or cancer-causing chemicals. The cause of most primary tumors remains a mystery.

The first test to diagnose brain and spinal column tumors is a neurological examination. Special imaging techniques (computed tomography, and magnetic resonance imaging, positron emission tomography) are also employed. Laboratory tests include the EEG and the spinal tap. A biopsy, a surgical procedure in which a sample of tissue is taken from a suspected tumor, helps doctors diagnose the type of tumor.

Tumors are classified according to the kind of cell from which the tumor seems to originate. The most common primary brain tumor in adults comes from cells in the brain called astrocytes that make up the blood-brain barrier and contribute to the nutrition of the central nervous system. These tumors are called gliomas (astrocytoma, anaplastic astrocytoma, or glioblastoma multiforme) and account for 65% of all primary central nervous system tumors. Some of the tumors are, but not limited to, Oligodendroglioma, Ependymoma, Meningioma, Lymphoma, Schwannoma, and Medulloblastoma.

### Neuroepithelial Tumors of the CNS

Astrocytic tumors, such as astrocytoma,; anaplastic (malignant) astrocytoma, such as hemispheric, diencephalic, optic, brain stem, cerebellar; glioblastoma multiforme; pilocytic astrocytoma, such as hemispheric, diencephalic, optic, brain stem, cerebellar; subependymal giant cell astrocytoma; and pleomorphic xanthoastrocytoma. Oligodendroglial tumors, such as oligodendroglioma; and anaplastic (malignant) oligodendroglioma. Ependymal cell tumors, such as ependymoma,; anaplastic ependymoma; myxopapillary ependymoma; and subependymoma. Mixed gliomas, such as mixed oligoastrocytoma; anaplastic (malignant) oligoastrocytoma; and others (e.g. ependymo-astrocytomas). Neuroepithelial tumors of uncertain origin, such as polar spongioblastoma; astroblastoma; and gliomatosis cerebri. Tumors of the choroid plexus, such as choroid plexus papilloma; and choroid plexus carcinoma (anaplastic choroid plexus papilloma). Neuronal and mixed neuronal-glial tumors, such as gangliocytoma; dysplastic gangliocytoma of cerebellum (Lhermitte-Duclos); ganglioglioma; anaplastic (malignant) ganglioglioma; desmoplastic infantile ganglioglioma, such as desmoplastic infantile astrocytoma; central neurocytoma; dysembryoplastic neuroepithelial tumor; olfactory neuroblastoma (esthesioneuroblastoma. Pineal Parenchyma Tumors, such as pineocytoma; pineoblastoma; and mixed pineocytoma/pineoblastoma. Tumors with neuroblastic or glioblastic elements (embryonal tumors), such as medulloepithelioma; primitive neuroectodermal tumors with multipotent differentiation, such as medulloblastoma; cerebral primitive neuroectodermal tumor; neuroblastoma; retinoblastoma; and ependymoblastoma.

### Other CNS Neoplasms

Tumors of the Sellar Region, such as pituitary adenoma; pituitary carcinoma; and craniopharyngioma. Hematopoietic tumors, such as primary malignant lymphomas; plasmacytoma; and granulocytic sarcoma. Germ Cell Tumors, such as germinoma; embryonal carcinoma; yolk sac tumor (endodermal sinus tumor); choriocarcinoma; teratoma; and mixed germ cell tumors. Tumors of the Meninges, such as meningioma; atypical meningioma; and anaplastic (malignant) meningioma. Non-menigothelial tumors of the meninges, such as Benign Mesenchymal; Malignant Mesenchymal; Primary Melanocytic Lesions; Hemopoietic Neoplasms; and Tumors of Uncertain Histogenesis, such as hemangioblastoma (capillary hemangioblastoma). Tumors of Cranial and Spinal Nerves, such as schwannoma (neurinoma, neurilemoma); neurofibroma; malignant peripheral nerve sheath tumor (malignant schwannoma), such as epithelioid, divergent mesenchymal or epithelial differentiation, and melanotic. Local Extensions from Regional Tumors; such as paraganglioma (chemodectoma); chordoma; chodroma; chondrosarcoma; and carcinoma. Metastatic tumours, Unclassified Tumors and Cysts and Tumor-like Lesions, such as Rathke cleft cyst; Epidermoid; dermoid; colloid cyst of the third ventricle; enterogenous cyst; neuroglial cyst; granular cell tumor (choristoma, pituicytoma); hypothalamic neuronal hamartoma; nasal glial herterotopia; and plasma cell granuloma.

Chemotherapeutics available are, but not limited to, alkylating agents such as, Cyclophosphamide, Ifosphamide, Melphalan, Chlorambucil, BCNU, CCNU, Decarbazine, Procarbazine, Busulfan, and Thiotepa; antimetabolites such as, Mcthotraxate, 5-Fluorouracil, Cytarabine, Gemcitabine (Gemzar®), 6-mercaptopurine, 6-thioguanine, Fludarabine, and Cladribine; anthracyclins such as, daunorubicin. Doxorubicin, Idarubicin, Epirubicin and Mitoxantrone; antibiotics such as, Bleomycin; camptothecins such as, irinotecan and topotecan; taxanes such as, paclitaxel and docetaxel; and platinums such as, Cisplatin, carboplatin, and Oxaliplatin.

The treatments are surgery, radiation therapy, immunotherapy, hyperthermia, gene therapy, chemotherapy, and combination of radiation and chemotherapy. Doctors also may prescribe steroids to reduce the swelling inside the CNS.

The compositions provided by the invention can provide a beneficial effect for adult cervical cancer, by administration of the nitrobenzamide compound or a combination of administration of the nitrobenzamide compound and radiation therapy, chemotherapy, or a combination thereof.

### PNS Cancers

The peripheral nervous system consists of the nerves that branch out from the brain and spinal cord. These nerves form the communication network between the CNS and the body parts. The peripheral nervous system is further subdivided into the somatic nervous system and the autonomic nervous system. The somatic nervous system consists of nerves that go to the skin and muscles and is involved in conscious activities. The autonomic nervous system consists of nerves that connect the CNS to the visceral organs such as the heart, stomach, and intestines. It mediates unconscious activities.

Acoustic neuromas are benign fibrous growths that arise from the balance nerve, also called the eighth cranial nerve or vestibulocochlear nerve. These tumors are non-malignant, meaning that they do not spread or metastasize to other parts of the body. The location of these tumors is deep inside the skull, adjacent to vital brain centers in the brain stem. As the tumors enlarge, they involve surrounding structures which have to do with vital functions. In the majority of cases, these tumors grow slowly over a period of years.

The malignant peripheral nerve sheath tumor (MPNST) is the malignant counterpart to benign soft tissue tumors such as neurofibromas and schwannomas. It is most common in the deep soft tissue, usually in close proximity of a nerve trunk. The most common sites include the sciatic nerve, brachial plexus, and sarcal plexus. The most common symptom is pain which usually prompts a biopsy. It is a rare, aggressive, and lethal orbital neoplasm that usually arises from sensory branches of the trigeminal nerve in adults. Malignant PNS tumor spreads along nerves to involve the brain, and most patients die within 5 years of clinical diagnosis. The MPNST may be classified into three major categories with epithelioid, mesenchymal or glandular characteristics. Some of the MPNST include but not limited to, Subcutaneous malignant epithelioid schwannoma with cartilaginous differentiation, Glandular malignant schwannoma, Malignant peripheral nerve sheath tumor with perineurial differentiation, Cutaneous epithelioid malignant nerve sheath tumor with rhabdoid features, Superficial epithelioid MPNST, Triton Tumor (MPNST with rhabdomyoblastic differentiation), Schwannoma with rhabdomyoblastic differentiation. Rare MPNST cases contain multiple sarcomatous tissue types, especially osteosarcoma, chondrosarcoma and angiosarcoma. These have sometimes been indistinguishable from the malignant mesenchymoma of soft tissue.

Other types of PNS cancers include but not limited to, malignant fibrous cytoma, malignant fibrous histiocytoma, malignant meningioma, malignant mesothelioma, and malignant mixed Mullerian tumor.

The treatments are surgery, radiation therapy, immunotherapy, chemotherapy, and combination of radiation and chemotherapy.

The compositions provided by the invention can provide a beneficial effect for PNS cancers, by administration of the nitrobenzamide compound or a combination of administration of the nitrobenzamide compound and radiation therapy, chemotherapy, or a combination thereof.

### Oral Cavity and Oropharyngeal Cancer

Management of patients with central nervous system (CNS) cancers remains a formidable task. Cancers such as, hypopharyngeal cancer, laryngeal cancer, nasopharyngeal cancer, oropharyngeal cancer, and the like, have been treated with surgery, immunotherapy, chemotherapy, combination of chemotherapy and radiation therapy. Etoposide and actinomycin D, two commonly used oncology agents that inhibit topoisomerase II, fail to cross the blood-brain barrier in useful amounts.

The compositions provided by the invention can provide a beneficial effect for Oral Cavity and Oropharyngeal cancer, by administration of the nitrobenzamide compound or a combination of administration of the nitrobenzamide compound and radiation therapy, chemotherapy, or a combination thereof.

### Stomach Cancer

Stomach cancer is the result of cell changes in the lining of the stomach. There are three main types of stomach cancers: lymphomas, gastric stromal tumors, and carcinoid tumors. Lymphomas are cancers of the immune system tissue that are sometimes found in the wall of the stomach. Gastric stromal tumors develop from the tissue of the stomach wall. Carcinoid tumors are tumors of hormone-producing cells of the stomach.

The causes of stomach cancer continue to be debated. A combination of heredity and environment (diet, smoking, etc) are all thought to play a part. Common approaches to the treatment include surgery, immunotherapy, chemotherapy, radiation therapy, combination of chemotherapy and radiation therapy or biological therapy.

The compositions provided by the invention can provide a beneficial effect for stomach cancer, by administration of the nitrobenzamide compound or a combination of administration of the nitrobenzamide compound and radiation therapy, chemotherapy, or a combination thereof.

### Testicular cancer

Testicular cancer is cancer that typically develops in one or both testicles in young men. Cancers of the testicle develop in certain cells known as germ cells. The 2 main types of germ cell tumors (GCTs) that occur in men are seminomas (60%) and nonseminomas (40%). Tumors can also arise in the supportive and hormone-producing tissues, or stroma, of the testicles. Such tumors are known as gonadal stromal tumors. The 2 main types are Leydig cell tumors and Sertoli cell tumors. Secondary testicular tumors are those that start in another organ and then spread to the testicle. Lymphoma is the most common secondary testicular cancer.

Common approaches to the treatment include surgery, immunotherapy, chemotherapy, radiation therapy, combination of chemotherapy and radiation therapy or biological therapy. Several drugs are typically used to treat testicular cancer: Platinol (cisplatin), Vepesid or VP-16 (etoposide) and Blenoxane (bleomycin sulfate). Additionally, Ifex (ifosfamide), Velban (vinblastine sulfate) and others may be used.

The compositions provided by the invention can provide a beneficial effect for stomach cancer, by administration of the nitrobenzamide compound or a combination of administration of the nitrobenzamide compound and radiation therapy, chemotherapy, or a combination thereof.

### Thymus Cancer

The thymus is a small organ located in the upper/front portion of your chest, extending from the base of the throat to the front of the heart. The thymus contains 2 main types of cells, thymic epithelial cells and lymphocytes. Thymic epithelial cells can give origin to thymomas and thymic carcinomas. Lymphocytes, whether in the thymus or in the lymph nodes, can become malignant and develop into cancers called Hodgkin disease and non-Hodgkin lymphomas. The thymus also contains another much less common type of cells called Kulchitsky cells, or neuroendocrine cells, which normally release certain hormones. These cells can give rise to cancers, called carcinoids or carcinoid tumors that often release the same type of hormones, and are similar to other tumors arising from neuroendocrine cells elsewhere in the body.

Common approaches to the treatment include surgery, immunotherapy, chemotherapy, radiation therapy, combination of chemotherapy and radiation therapy or biological therapy. Anticancer drugs that have been used in the treatment of thymomas and thymic carcinomas are doxorubicin (adriamycin), cisplatin, ifosfamide, and corticosteroids (prednisone). Often, these drugs are given in combination to increase their effectiveness. Combinations used to treat thymic cancer include cisplatin, doxorubicin, etoposide and cyclophosphamide, and the combination of cisplatin, doxorubicin, cyclophosphamide, and vincristine.

The compositions provided by the invention can provide a beneficial effect for stomach cancer, by administration of the nitrobenzamide compound or a combination of administration of the nitrobenzamide compound and radiation therapy, chemotherapy, or a combination thereof.

### EXAMPLES

### Example 1

### Baseline solubility of 4-iodo-3-nitrobenzamide in water, acid, base, and sodium chloride

Excess 4-iodo-3-nitrobenzamide ("BA") was equilibrated overnight (>16 hr) at 25° C in purified water, 0.0 1 M HCl (pH2), 0.01M NaOH (pH 13), and 0.9% NaCl. Following dilution, the solubility of the drug was measured by HPLC. The drug solubility results are shown in Table 1.

**Table 1**

| **Media** | **Solubility (mg/ml)** |
|---|---|
| Purified Water | 0.182 |
| 0.01 M HCl (pH 2) | 0.179 |
| 0.01M NaOH (pH 13) | 0.181 |
| 0.9% NaCl | 0.164 |
| 5% Glucose (5GW) | 0.173 |

As can be seen from Table 1, the solubility of BA in water without any solubilizer is ≤ 0.2 mg/ml. For purposes of calculating enhancement of solubility of BA in water, 0.2 mg/ml is taken as the baseline solubility of BA, against which various solubilizers are tested.

### Example 2

### Solubility of 4-iodo-3-nitrobenzamide in water with cyclodextrin

Excess 4-iodo-3-nitrobenzamide was equilibrated overnight (>16 hr) at 25° C in solutions of purified water containing various concentrations of cyclodextrins including hydroxypropyl-β-cyclodextrin (Kleptose® from Roquette) (HPBCD), ±sulfobutyl ether- β-cyctodextrin (Captisol® from Cydex) (SBEBCD), and Hyroxypropyl-γ-cyclodextrin (Cavamax W8® from Wacker) (HPGCD). Following dilution, the solubility of the drug was measured by HPLC. The drug solubility results are shown in Table 2. A plot of drug solubility versus concentration of HPBCD is shown in Figure 1.

**Table 2**

| **Solubilizer** | **Level** | **Solubility (mg/ml)** | **Enhancement** |
|---|---|---|---|
| None | 0% | 0.182 | 1.0 |
| HPBCD | 10% | 4.860 | 26.7 |
| HPBCD | 20% | 9.965 | 54.8 |
| HPBCD | 25% | 11.465 | 63.0 |
| HPBCD | 40% | 12.769 | 70.2 |
| HPGCD | 25% | 0.272 | 1.5 |
| SBEBCD | 25% | 11.036 | 60.6 |

### Example 3

### FORMULATION STUDIES

Addition ofTonicity Adjusters: Initial studies on 10 ml volumes of solution investigated the use of sodium chloride as a tonicity adjuster since it was felt that its inclusion may reduce the level of hemolysis associated with the use of hydroxypropyl-β-cyclodextrin (HPβCD).

Osmolality Measurements: Osmolality determinations where carried out using a Roebling Freezing Point Depression Osmometer.

Since the osmolality of a 25% HPβCD solution was already 249 mOsmol/kg addition of tonicity adjusters results in the formation of a hypertonic solution (see Table 6). However addition of small quantities of tonicity adjuster may be employed to reduce the level of chemical lysis associated with some formulation excipients. Solutions that are slightly hypertonic are usually satisfactorily tolerated on intravenous administration.

pH Determination: The pH of formulations was measured to determine if these would be suitable to provide a well-tolerated formulation.

Compounding Studies (30 ml or 50 ml lots) are summarized in Table 6:

**Table 6: Formula**

| **Material** | **Percentage Formula** |
|---|---|
| BA | 1.0% w/v |
| Kleptose HPB | 25%w/v |
| Sodium Chloride | 0.6% w/v |
| Purified water | to 100% v/v |

Two trials were carried out:
1. 300 mg ofBA was added to 30 ml of a 25% HPBCD (containing 0.6% saline) and mixed using a magnetic stirrer. The drug substance dissolved in 4 hours. This is deemed to be an unacceptably long time for a viable manufacturing process and it was decided to evaluate a procedure involving dissolving the drug substance directly in the HPBCD.
2. In a second study 500 mg of BA dissolved in a 50 ml of a 25% HPBCD vehicle (no saline) in approx 90 minutes. The dissolution rate of BA was deemed to be a more acceptable time and provides some prospect for a viable larger scale process. Further excipients could be added after the drug substance has been dissolved.

Taken together these observations suggest that saline may reduce the (kinetic) drug solubility possibly as a consequence of the ionic strength. Further evidence of a reduction in solubility in the presence of saline was obtained from the filtration study (below).

Filtration: The solutions described above could be satisfactorily filtered through a 0.2 µm syringe filter.

Surprisingly when solution #2 described above was filtered through a 0.2 µm PVDF disk filter precipitation occurred. This suggests that the inclusion of saline has resulted in reduction in BA solubility.

Alternative tonicity adjusters (e.g. glucose and buffers) were therefore investigated.

Tonicity adjustment with Glucose (10 ml - 25 ml volumes): Solutions of BA in 25% HPBCD were prepared containing various levels of glucose and 10 mM buffer (disodium hydrogen phosphate dodecahydrate, sodium dihydrogen phosphate dihydrate). The pH and Osmolality were determined as described previously. Results are provided in Table 7.

Solutions (approx 25 ml volumes) of the glucose and phosphate buffered solutions could be satisfactorily filtered through a 0.2 µm PVDF syringe filter.

The use of a phosphate buffer system may provide an advantage compared to an unbuffered system since a more stable pH is obtained. A further advantage is that the 10 mM phosphate buffer level provides a formulation that is virtually isotonic with blood.

### Hemolysis Studies

Summary of Procedure: The procedure involves adding 1 ml of sheep's blood to a sample (0.1 ml and 0.25 ml) of the formulation. The samples are vortex mixed and diluted with 0.9% saline, mixed for a further time and then centrifuged (1500 r.p.m.) and observed for evidence of a red color indicating the presence of hemoglobin caused by lysis. 0.1 ml of the supernatant is diluted with 2 ml of 0.9% saline and centrifuged for a further 5 minutes. The solution is then compared to a positive control (0.9% saline providing no hemolysis) and a negative control (water to provide complete hemolysis).

Results: None of the phosphate buffered or glucose adjusted solutions demonstrated evidence of hemolysis. Although a very faint pink tinge was observed in the samples, the intensity was similar to the positive saline control. During use of this procedure it has been noted that the lot of sheep's blood used can influence the degree of color in the positive control.

**Table 7: Summary of Results for Glucose and Phosphate Buffered Systems**

| Vehicle | Osmolality (mOsmol/kg) | pH | Hemolysis |
|---|---|---|---|
| 25% HPβCD (no tonicity adjusters or buffers) | 249 | 8.54 | N |
| 25% HPβCD + 2.5% glucose + no buffer | 466 | 8.03 | N |
| 25% HPβCD + 5% glucose + no buffer | 703 | 8.18 | N |
| 25% HPβCD + no glucose + 10 mM phosphate buffer | 296 | 7.45 | N |
| 25% HPβCD + 2.5% glucose + 10 mM phosphate buffer | 503 | 7.44 | N |
| 25% HPβCD + 5% glucose + 10 mM phosphate buffer | 746 | 7.40 | N |
| 25% HPβCD + 0.45% saline | 617 | ND | ND |
| 25% HPβCD + 0.6% saline | 494 | ND | ND |
| 25% HPβCD + 0.9% saline | 430 | ND | ND |

| | | | |
|---|---|---|---|
| Shaded region is currently the preferred formulation. ND = Not Determined (precipitation on filtration of saline solutions). | | | |

Conclusion: The 10 mM phosphate buffered solution is especially interesting, as: (a) The pH is similar to that of blood; (b) The pH of solutions should remain more stable on storage; (c) The osmotic pressure of the phosphate buffered formulation (296 mOsmol/kg) is similar to blood; (d) There is no need to include a separate tonicity adjuster to achieve isotonicity; (e) The phosphate buffered formulations without tonicity adjusters appear to be filterable without risk of precipitation; (t) The use of saline (causing precipitation) and glucose (potentially decomposing to form 5-HMF on autoclaving) could be avoided; It has been reported that Phosphate is allegedly not complexed by CDs hence solubility should not be affected.

## Claims

1. A composition in aqueous solution form comprising 4-iodo-3-nitrobenzamide or a pharmaceutically acceptable salt thereof: a cyclodextrin which is hydroxypropyl-β-cyclodextrin or sulphobutyl ether-β-cyctodextrin, and a physiologically compatible buffer for injection, for use in the treatment of a cancer or a viral condition.

2. A composition as claimed in Claim 1, wherein the cancer is a member of the group consisting of leukemia, breast cancer, ovarian cancer, lung cancer, bladder cancer, prostate cancer, pancreatic cancer, and cervical cancer.

3. A composition as claimed in any preceding claim wherein the cancer is a member of the group consisting of breast cancer, ovarian cancer, lung cancer, and pancreatic cancer.

4. A composition as claimed in any preceding claim wherein the cancer is breast cancer.

5. A composition as claimed in any one of Claims 1 to 3 wherein the cancer is ovarian cancer.

6. A composition as clamed in any one of Claims 1 to 3 wherein the cancer is lung cancer.

7. A composition as claimed in any one of Claims 1 to 3 wherein the cancer is pancreatic cancer.

8. A composition in aqueous solution form for injection comprising 4-iodo-3-nitro-benzamide or a pharmaceutically acceptable salt thereof, a physiologically compatible buffer, and a cyclodextrin which is hydroxypropyl-β-cyclodextrin or sulphobutyl ether-β-cyclodextrin.

9. A composition for use according to Claim 1 or a composition as claimed in Claim 8, wherein the cyclodextrin is present in said aqueous solution at a concentration of from 25% to 40% (w/v) of the composition.

10. A composition for use according to Claim 1 or a composition as claimed in Claim 8, wherein the cyclodextrin is present in said aqueous solution at a concentration of 25% or 40% (w/v) of the composition.

11. A composition for use according to Claim 1 or a composition as claimed in Claim 8, wherein the cyclodextrin is hydroxypropyl-β-cyclodextrin.

12. A composition for use according to Claim 1 or a composition as claimed in Claim 8. wherein the Cyclodextrin is 2-hydroxypropyl-β-cyclodextrin.

13. A composition for use according to Claim 1 or a composition as claimed in Claim 8, wherein the cyclodextrin is Kleptose^{™}.

14. A composition for use according to Claim 1 or a composition as claimed in Claim 8, wherein the cyclodextrin is present at a concentration of 25% (w/v).

15. A composition for use according to Claim 1 or a composition as claimed in Claim 8, wherein the cyclodextrin is present at a concentration of 25% and the buffer is 10 mM phosphate at pH 7.4.

16. A composition for use according to Claim 1 or a composition as claimed in Claim 8. wherein the weight ratio of the cyclodextrin to 4-iodo-3-nitro-benzamide is from 1:100 to 5000: 1.

17. A composition for use according to Claim 1, wherein the cyclodextrin is 2-hydroxypropyl-β-cyclodextrin and is present at a concentration of 25%, and the buffer is 10 mM phosphate at pH 7.4.

18. A composition as claimed in Claim 8, wherein the cyclodextrin is 2-hydroxypropyl-β-cyclodextrin and is present at a concentration of 25%, and the buffer is 10 mM phosphate at pH 7.4.

## Patentansprüche

1. Zusammensetzung in Form einer wässrigen Lösung, welche 4-Iod-3-nitrobenzamid oder ein pharmazeutisch unbedenkliches Salz davon, ein Cyclodextrin, bei dem es sich um Hydroxypropyl-β-Cyclodextrin oder Sulfobutylether-β-cyclodextrin handelt, und einen physiologisch verträglichen Puffer für Injektionszwecke enthält, zur Verwendung bei der Behandlung eines Krebs- oder Virenleidens.

2. Zusammensetzung nach Anspruch 1, wobei es sich bei dem Krebs um ein Mitglied der aus Leukämie, Brustkrebs, Eierstockkrebs, Lungenkrebs, Blasenkrebs, Prostatakrebs, Bauchspeicheldrüsenkrebs und Gebärmutterhalskrebs bestehenden Gruppe handelt.

3. Zusammensetzung nach Anspruch 1 oder 2, wobei es sich bei dem Krebs um ein Mitglied der aus Brustkrebs, Eierstockkrebs, Lungenkrebs und Bauchspeicheldrüsenkrebs bestehenden Gruppe handelt.

4. Zusammensetzung nach einem der vorhergehenden Ansprüche, wobei es sich bei dem Krebs um Brustkrebs handelt.

5. Zusammensetzung nach einem der Ansprüche 1 bis 3, wobei es sich bei dem Krebs um Eierstockkrebs handelt.

6. Zusammensetzung nach einem der Ansprüche 1 bis 3, wobei es sich bei dem Krebs um Lungenkrebs handelt.

7. Zusammensetzung nach einem der Ansprüche 1 bis 3, wobei es sich bei dem Krebs um Bauchspeicheldrüsenkrebs handelt.

8. Zusammensetzung in Form einer wässrigen Lösung für Injektionszwecke, welche 4-Iod-3-nitrobenzamid oder ein pharmazeutisch unbedenkliches Salz davon, einen physiologisch verträglichen Puffer und ein Cyclodextrin, bei dem es sich um Hydroxypropyl-β-Cyclodextrin oder Sulfobutylether-β-Cyclodextrin handelt, enthält.

9. Zusammensetzung zur Verwendung nach Anspruch 1 oder Zusammensetzung nach Anspruch 8, wobei das Cyclodextrin in der wässrigen Lösung in einer Konzentration von 25% bis 40% (w/v) der Zusammensetzung vorliegt.

10. Zusammensetzung zur Verwendung nach Anspruch 1 oder Zusammensetzung nach Anspruch 8, wobei das Cyclodextrin in der wässrigen Lösung in einer Konzentration von 25% oder 40% (w/v) der Zusammensetzung vorliegt.

11. Zusammensetzung zur Verwendung nach Anspruch 1 oder Zusammensetzung nach Anspruch 8, wobei es sich bei dem Cyclodextrin um Hydroxypropyl-β-Cyclodextrin handelt.

12. Zusammensetzung zur Verwendung nach Anspruch 1 oder Zusammensetzung nach Anspruch 8, wobei es sich bei dem Cyclodextrin um 2-Hydroxypropyl-β-Cyclodextrin handelt.

13. Zusammensetzung zur Verwendung nach Anspruch 1 oder Zusammensetzung nach Anspruch 8, wobei es sich bei dem Cyclodextrin um Kleptose^{™} handelt.

14. Zusammensetzung zur Verwendung nach Anspruch 1 oder Zusammensetzung nach Anspruch 8, wobei das Cyclodextrin in einer Konzentration von 25% (w/v) vorliegt.

15. Zusammensetzung zur Verwendung nach Anspruch 1 oder Zusammensetzung nach Anspruch 8, wobei das Cyclodextrin in einer Konzentration von 25% vorliegt und es sich bei dem Puffer um 10 mM Phosphat mit einem pH-Wert von 7,4 handelt.

16. Zusammensetzung zur Verwendung nach Anspruch 1 oder Zusammensetzung nach Anspruch 8, wobei das Gewichtsverhältnis des Cyclodextrins zum 4-Iod-3-nitrobenzamid 1:100 bis 5000:1 beträgt.

17. Zusammensetzung zur Verwendung nach Anspruch 1, wobei es sich bei dem Cyclodextrin um 2-Hydroxypropyl-β-Cyclodextrin handelt, welches in einer Konzentration von 25% vorliegt, und es sich bei dem Puffer um 10 mM Phosphat mit einem pH-Wert von 7,4 handelt.

18. Zusammensetzung nach Anspruch 8, wobei es sich bei dem Cyclodextrin um 2-Hydroxypropyl-β-Cyclodextrin handelt, welches in einer Konzentration von 25% vorliegt, und es sich bei dem Puffer um 10 mM Phosphat mit einem pH-Wert von 7,4 handelt.

## Revendications

1. Composition sous forme de solution aqueuse comprenant du 4-iodo-3-nitrobenzamide ou un sel pharmaceutiquement acceptable de celui-ci ; une cyclodextrine qui est l'hydroxypropyl-β-cyclodextrine ou la sulfobutyléther-β-cyclodextrine, et un tampon physiologiquement compatible pour injection, destinée à être utilisée dans le traitement d'un cancer ou d'une affection virale.

2. Composition selon la revendication 1, **caractérisée en ce que** le cancer est un membre du groupe constitué par une leucémie, le cancer du sein, le cancer de l'ovaire, le cancer du poumon, le cancer de la vessie, le cancer de la prostate, le cancer du pancréas et le cancer du col de l'utérus.

3. Composition selon l'une ou l'autre des revendications précédentes, **caractérisée en ce que** le cancer est un membre du groupe constitué par le cancer du sein, le cancer de l'ovaire, le cancer du poumon et le cancer du pancréas.

4. Composition selon l'une quelconque des revendications précédentes, **caractérisée en ce que** le cancer est le cancer du sein.

5. Composition selon l'une quelconque des revendications 1 à 3, **caractérisée en ce que** le cancer est le cancer de l'ovaire.

6. Composition selon l'une quelconque des revendications 1 à 3, **caractérisée en ce que** le cancer est le cancer du poumon.

7. Composition selon l'une quelconque des revendications 1 à 3, **caractérisée en ce que** le cancer est le cancer du pancréas.

8. Composition sous forme de solution aqueuse pour injection comprenant du 4-iodo-3-nitrobenzamide ou un sel pharmaceutiquement acceptable de celui-ci, un tampon physiologiquement compatible, et une cyclodextrine qui est l'hydroxypropyl-β-cyclodextrine ou la sulfobutyléther-β-cyclodextrine.

9. Composition destinée à être utilisée selon la revendication 1 ou une composition selon la revendication 8, **caractérisée en ce que** la cyclodextrine est présente dans ladite solution aqueuse à une concentration de 25 % à 40 % (p/v) de la composition.

10. Composition destinée à être utilisée selon la revendication 1 ou une composition selon la revendication 8, **caractérisée en ce que** la cyclodextrine est présente dans ladite solution aqueuse à une concentration de 25 % ou 40 % (p/v) de la composition.

11. Composition destinée à être utilisée selon la revendication 1 ou une composition selon la revendication 8, **caractérisée en ce que** la cyclodextrine est l'hydroxypropyl-β-cyclodextrine.

12. Composition destinée à être utilisée selon la revendication 1 ou une composition selon la revendication 8, **caractérisée en ce que** la cyclodextrine est la 2-hydroxypropyl-β-cyclodextrine.

13. Composition destinée à être utilisée selon la revendication 1 ou une composition selon la revendication 8, **caractérisée en ce que** la cyclodextrine est Kleptose^{™}.

14. Composition destinée à être utilisée selon la revendication 1 ou une composition selon la revendication 8, **caractérisée en ce que** la cyclodextrine est présente à une concentration de 25 % (p/v).

15. Composition destinée à être utilisée selon la revendication 1 ou une composition selon la revendication 8, **caractérisée en ce que** la cyclodextrine est présente à une concentration de 25 % et le tampon est du phosphate 10 mM à pH 7,4.

16. Composition destinée à être utilisée selon la revendication 1 ou une composition selon la revendication 8, **caractérisée en ce que** le rapport en poids entre la cyclodextrine et le 4-iodo-3-nitrobenzamide est de 1:100 à 5000:1.

17. Composition destinée à être utilisée selon la revendication 1, **caractérisée en ce que** la cyclodextrine est la 2-hydroxypropyl-β-cyclodextrine et est présente à une concentration de 25 %, et le tampon est du phosphate 10 mM à pH 7,4.

18. Composition selon la revendication 8, **caractérisée en ce que** la cyclodextrine est la 2-hydroxypropyl-β-cyclodextrine et est présente à une concentration de 25 %, et le tampon est du phosphate 10 mM à pH 7,4.
